Europäisches Patentamt

European Patent Office

Office européen des brevets

(11) Veröffentlichungsnummer: **0 100 447**
**A2**

(12) **EUROPÄISCHE PATENTANMELDUNG**

(21) Anmeldenummer: 83106452.2

(22) Anmeldetag: 01.07.83

(51) Int. Cl.³: **A 61 K 7/06**
**A 61 K 7/13**

(30) Priorität: 02.07.82 HU 218382

(43) Veröffentlichungstag der Anmeldung:
15.02.84 Patentblatt 84/7

(84) Benannte Vertragsstaaten:
AT CH DE FR GB IT LI SE

(71) Anmelder: Gouth, János, Dr.
Sziklai Sándor u. 66
H-2045 Törökbálint(HU)

(72) Erfinder: Gouth, János, Dr.
Sziklai Sándor u. 66
H-2045 Törökbálint(HU)

(74) Vertreter: Weickmann, Heinrich, Dipl.-Ing. et al,
Patentanwälte Dipl.-Ing. H.Weickmann Dipl.-Phys.Dr. K.
Fincke Dipl.-Ing. F.A. Weickmann Dipl.-Chem. B. Huber
Dr.-Ing. H. Liska Dipl.-Phys.Dr. J. Prechtel Möhlstrasse
22
D-8000 München 86(DE)

(54) Kosmetisches Mittel von haarregenerierender Wirkung.

(57) Ein kosmetisches Präparat von haarregenerierender Wirkung, das eine in Alkohol zubereitete Lösung von 0,11 bis 0,20 Gew.% Perubalsam, 1,12 bis 1,85 Gew.% Schwefel und 11,0 bis 14,55 Gew.% Glyzerin, ferner in ionfreiem Wasser gelöst 1,28 bis 1,54 Gew.% Ammoniumchlorid, 0,133 bis 1,33 Gew.% basische Bleiazetatlösung und/oder 0,133 bis 0,666 Gew.% Aluminiumazetat sowie 0,566 bis 0,766 Gew.% mit Laurinsäure ästherifizierte Polyäthylen-Glykolästher von Sorbitanhydriden enthält, wobei im Präparat in erwünschten Falle noch Pantothensäure und als Tragstoff ionfreies Wasser, sowie gegebenenfalls auch weitere, im kosmetischen Haarcremen üblicherweise verwendete Zusatzstoffe mit enthalten sind.

EP 0 100 447 A2

# Kosmetisches Mittel von haarregenerierender Wirkung

Die Erfindung betrifft ein zur Verhinderung des Haarergrauens, zum Aufhalten des Ergrauungsprozesses, sowie zur Rückfärbung des grau gewordenen Haars geeignetes kosmetisches Mittel von haarregenerierender Wirkung.

Es ist wohlbekannt, dass man seit langer Zeit danach bestrebt ist, den Ergrauungsprozess aufhalten zu können, bzw. zur Rückfärbung der Haare dienende, farbstofffreie Präparate zur erzeugen.

Der genaue Mechanismus des Haarergrauens – im Laufe dessen die Farbstoffproduktionsfähigkeit der Haarzwiebel aufhört – ist noch nicht bekannt. Gemäss Vermutungen kann es auf zahlreiche Vorereignisse, bzw. den Mechanismus auslösende Gründe zurückgeführt werden. Derartige auslösende Gründe z.B. Krankheit, Heredität oder mit dem Nervensystem zusammenhängende Probleme usw. sein. Die Rindensubstanz der grau gewordenen Haare ist frei von Farbstoffen, und wenn die Marksubstanz erhalten blieb, ist das Haar weiss. /Es gibt Hypothesen, wonach die weisse Farbe auf die im Mark akkumulierte Luft zurückzuführen ist./ Meistens ist das graue Haar brüchiger und von minderer Qualität, als das aus den Pigment produzierenden Zwiebeln gewachsene normale Haar.

0100447

In aller Welt wird das Haarergrauen in der Mehrheit der Fälle durch Färben eliminiert. Selbstverständlich wird aber das graue Haar beim Haarwachsen wieder und wieder ersichtlich. Die Färbung ist nicht nur kostaufwendig, sondern die meisten Farbstoffe sind für das Haar gefährlich, und sie gefährden auch die Haarzwiebel und die Kopfhaut.

Zur Verzögerung des Haarergrauens wurde versucht, Pantothensäure, Paraaminobezoesäure /Vitamin H/ und Kalziumpantothenat B-Komplex zu verwenden. Bei Tierversuchen konnte das Fabstoffgehalt der Haare durch Zufuhr von Cu und Fe erhöht werden. Ein das Problem ohne Färbung vollkommen lösendes und allgemein wirksames Präparat oder eine derartige Kur konnten jedoch bisher nicht erfolgreich entwickelt werden.

Der Erfindung wurde das Ziel gesetzt, unter Beseitigung der obenerwähnten Mängel ein von jedwelchem Farbstoff freies, den Prozess des Haarergrauens aufhaltendes, bzw. ein die Möglichkeit der Rückfärbung der grauen Haare anbietendes Präparat zu entwickeln.

- Im Sinne der Erfindung wurde ein kosmetisches Präparat ausgearbeitet, bei dessen Anwendung bereits eine einzige Kur die ursprüngliche Haarfarbe zurückgibt, und bei regelmässigem Gebrauch dieser Zustand aufrechterhalten bleibt, während das regenerierende Präparat die Haare verstärkt, ja sogar eine den Haarausfall vermindernde, ausdrücklich haarregenerierende Wirkung ausübt.

Das erfindungsgemässe Präparat stellt eine Kombination dar, deren Bestandteile - wenn in einer vorbestimmten Menge und gemeinsam verwendet - die gewünschte Wirkung gewährleisten. Das erfindungsgemässe Präparat ist dadurch gekennzeichnet, dass es in Alkohol gelösten Perubalsam, Schwefel und Glyzerin, Ammoniumchlorid, basisches Bleiazetat und /oder Aluminiumazetat, sowie die mit Laurinsäure gebildeten Polyäthylen-Glykoäster von Sorbitanhydriden - in ionfreiem Wasser gelöst -, desweiteren denen zugegebene Pantothensäure enthält.

Aus den obenerwähnten Komponenten sind für die Rückstellung der ursprünglichen Haarfarbe der Schwefel, das basische Bleiazetat und /oder Aluminiumazetat, sowie die Pantothensäure - wenn gemeinsam verwendet - von grösster Bedeutung. Nach unseren Beobachtungen ergänzen die weiteren Komponenten die erwähnte Wirkung, und sie üben dabei sonstige haarregenerierende Einflüsse aus.

Bei der erfindungsgemässen Komposition fanden wir es überraschend, dass es mit der Pantothensäure allein - obzwar diese, wie bereits erwähnt, zu diesem Zweck früher verwendet worden ist - keine befriedigenden Resultate erreicht werden konnten. Auch der Schwefel ist schon lange als Komponente von Haarpflegemitteln bekannt. Eine mit dem Haarergrauen zusammenhängende Wirkung konnte jedoch bisher nicht beobachtet werden. So kann es angenommen werden, dass im Falle der erfindungsgemässen Komposition eine gewisse Wechselwirkung entsteht, die sich aufgrund eines bisher unbekannten Mechanismus entfaltet. Für alle Fälle besteht jedoch die Tatsache, dass nach dem Ablauf von etwa einer Woche, bei einem täglichen Gebrauch die Haare ihre ursprüngliche Farbe zurückgewinnen, die Qualität der Haare sich verbessert, und dieser Zustand durch eine regelmässige Behandlung dauerhaft aufrechterhalten werden kann.

Wenn nun das erfindungsgemässe Präparat zu gebrauchen beabsichtigt wird, soll eine einwöchige Kur genommen werden, welche aus täglich zweimal vorzunehmenden Einreiben über eine Woche besteht. Die Behandlung ist so durchzuführen, dass man nach erfolgtem Aufschütteln des Flascheninhalts ein Stück Watte von gewünschter Grösse mit der Flüssigkeit durchtränkt, und die Flüssigkeit sofort in den ganzen Haarbestand des Kopfes einreibt. Das Einreiben soll nicht zu stark, aber ganz bis zu den Haarwurzeln dringend durchgeführt werden, sodass die Haare durchtränkt werden. Die Watte soll dabei mehrmals durchtränkt werden — wovor die Flasche stets gründlich zu schütteln ist — wonach der Kopf allmählich über die einzelnen Kopfteile gründlich mit der Flüssigkeit eingerieben wird.

Darauffolgend wird das Haar — um die Gefahr der Erkältung zu vermeiden — mit geringer Wärme getrocknet, oder mit einem Tuch bedeckt werden, wobei eine natürliche Trocknung ebenfalls zugelassen ist.

Im Laufe des Einreibens kleben die Haare geringfügig zusammen, so dass das Haar noch in feuchtem Zustand vorsichtig ausgekämmt werden soll.

Am neunten Tag, vom Beginn der Behandlung angerechnet, können die Haare mit jedwelchem Schampoo gewaschen werden.

Nach einer Wochenkur scheint es zweckmässig zu sein, die Behandlung wöchentlich einmal oder zweimal zu wiederholen.

Das erfindungsgemässe Präparat kann auch in Form einer kosmetischen Creme zubereitet bzw. auf den Markt gebracht werden. In diesem Fall werden die Wirkstoffe in weniger Lösungsmittel aufgelöst und den vereinigten Komponenten werden die an sich bekannten, in Haarpflegemitteln verwendeten Zusatzstoffe zugegeben.

Die erfindungsgemässe Lösung wird anhand Beispiele näher erläutert.

Beispiel 1

Es wird als Ziel gesetzt, ein Schüttelgemisch der Menge von 150ml zuzubereiten, das für eine in Form einer siebentägigen Kur vorzunehmenden Behandlung von ergrauten Haaren von durchschnittlicher Dichte und Menge ausreichend ist, und dabei die ursprüngliche Haarfarbe zurückgibt.

In 13,33 % 96%-igem Alkohol wird 0,13% Perubalsam aufgelöst. Nach Auflösen werden

0100447

1,33 % fein geriebener Schwefel - und unter ständiger Rührung - 13,33 % Glyzerin zugegeben.

Diesweiteren wird in 13,33% der Gesamtmenge entsprechendem ionfreiem Wasser Ammoniumchlorid in einer Menge von 1,33 %- und unter ständiger Rührung gelöst, und hierzu

1,33 % basische Bleiazetatlösung zugegeben.

Darauffolgend werden die in Alkohol gelöste und die wässerige Lösung gemischt, wonach der ganzen Lösung in einer Menge von 0,66% - auf die endgültige Menge bezogen - die mit Laurinsäure esterifizierten Polyäthylen-Glykol-ester von Sorbitenhydriden und zuletzt 1,33% Pantethensäure zugegeben werden.

Die auf diese Weise erhaltene Mischung wird mit ionfreiem Wasser auf 150 ml ergänzt, so bildet die zugegebene Wassermenge 54,66 % des Gemisches.

Beispiel 2

In jeder Hinsicht wird wie bei dem Beispiel 1 verfahren, jedoch mit dem Unterschied, dass die Menge der Bleiazetatlösung verringert, daneben Aluminiumazetat dem Gemisch zugegeben, und der Inhalt an Pantethensäure verdreifacht wird.

Demnach enthält die Komposition die folgenden Komponenten:

| | |
|---|---|
| Perubalsam | 0,133 % |
| pulverisierter Schwefel | 1,333 % |
| Glyzerin | 13,333 % |
| Ammoniumchlorid | 1,333 % |

0100447

| | |
|---|---|
| Basische Bleiazetatlösung | 0,133 % |
| Aluminiumazetat | 0,666 % |
| Die mit Laurinsäure gebildeten | |
| Polyäthylen-Glykolester von | |
| Sorbitanhydriden | 0,666 % |
| Pantothensäure | 3,999 % |
| Alkohol /96%-ig/ | 13,333 % |
| Ionfreies Wasser | 67,200 % |

0100447

Patentanspruch

Ein kosmetisches Präparat von haarregenerierender Wirkung,
d a d u r c h   g e k e n n z e i c h n e t, dass es eine
in Alkohol zubereitete Lösung von 0,11 bis 020 Gew.% Perubalsam, 1,12 bis 1,85 Gew.% Schwefel und 11,0 bis 14,55 Gew.%
Glyzerin, ferner in ionfreiem Wasser gelöst 1,28 bis 1,54 Gew.%
Ammoniumchlorid, 0,133 bis 1,33 Gew.% basische Bleiazetatlösung und/oder 0,133 bis 0,666 Gew.% Aluminiumazetat sowie
0,566 bis 0,766 Gew.% mit Laurinsäure ästherifizierte Polyäthylen-Glykolästher von Sorbitanhydriden enthält, wobei im
Präparat in erwünschten Falle noch Pantothensäure und als
Tragstoff ionfreies Wasser, sowie gegebenenfalls auch weitere,
im kosmetischen Haarcremen üblicherweise verwendete Zusatzstoffe mit enthalten sind.